# EUROPÄISCHE PATENTSCHRIFT

(11) **EP 0 452 276 B1**
(45) Veröffentlichungstag und Bekanntmachung des Hinweises auf die Patenterteilung: **23.08.1995**
(21) Anmeldenummer: 91810264.1
(22) Anmeldetag: 10.04.1991
(51) Int. Cl.: A61B 5/026

(54) **Blutflussmessgerät**
Apparatus for measuring blood flow
Dispositif de mesure de débit sanguin

(30) Priorität: 12.04.1990 CH 1277/90
(43) Veröffentlichungstag der Anmeldung: 16.10.1991
(73) Patentinhaber: Baer, Hans, Dr., CH-8006 Zürich (CH); Hirsbrunner, Eduard, Dr., CH-8006 Zürich (CH)
(72) Erfinder: Lienhard, Brigit, CH-8006 Zürich (CH)
(74) Vertreter: Ritscher, Thomas, Dr.

(56) Entgegenhaltungen:
- DE-A- 3 330 358
- IEEE TRANSACTIONS ON BIOMEDICAL ENGINEERING Band BME-28, Nr. 3, März1981, Seiten 265-270, New York, US; J.B. MYKLEBUST et al.: "A combination isothermal-hydrogen clearance system for the measurement of local tissue flow"
- PHYSICS IN MEDICINE & BIOLOGY Band 34, Nr. 10, Oktober 1989, Seiten 1413-1428, Woodbury, NY, US; D.K.HARRISON et al.: "Local hydrogen clearance as a method for the measurement of capillary blood flow"
- STROKE Band 11, Nr. 5, September/Oktober 1980, Seiten 552-564, New York, US; W. YOUNG: "H2 clearance measurement of blood flow: A review of technique and polarographic principles"

## Beschreibung

Die vorliegende Erfindung betrifft ein Blutflussmessgerät nach dem Wasserstoff-Auswasch-Verfahren mit einer Messelektrode, einer Referenzelektrode und einer Neutralelektrode, sowie mit einem Operationsverstärker und mit einer Signalanzeigevorrichtung.

Die Durchblutung des menschlichen und tierischen Gewebes ist ein wichtiger Indikator sowohl für die Diagnostik als auch für die Kontrolle des Verlaufs und Erfolgs einer Therapie oder eines chirurgischen Eingriffs sowie für das Erproben und Beobachten der Wirkung von Medikamenten. Es sind darum schon viele Publikationen, in denen über Erfahrungen mit Messgeräten dazu berichtet wird, bekannt.

Bei einem bekannten Verfahren zum Bestimmen der Durchblutung von Geweben wird ein geeignetes radioaktives Material mit kurzer Halbwertszeit in das zu untersuchende Gewebe implantiert. Die Zerfallsprodukte dieses radioaktiven Materials weisen vorzugsweise eine noch kürzere Halbwertszeit auf und sind als gasförmige Substanz im Blut löslich. Beim radioaktiven Zerfall des implantierten Materials und der Zerfallsprodukte entstehen Strahlungen, deren Intensität mit geeigneten Geräten gemessen wird. Das Verhältnis dieser Intensitäten bzw. die Aenderung dieses Verhältnisses mit der Zeit ermöglicht die quantitative Bestimmung der Menge der vom Blutfluss aus dem Gewebe ausgewaschenen Zerfallprodukte und damit auch die Bestimmung der Durchblutung des Gewebes.

Die Nachteile dieses Verfahrens sind offensichtlich. Es sind bisher nur wenig radioaktive Materialien mit den vorerwähnten Eigenschaften bekannt, die mit der erforderlichen Reinheit hergestellt werden können. Das Implantieren des radioaktiven Materials erfordert einen chirurgischen Eingriff und kann nicht an jedem Gewebe oder Organ vorgenommen werden. Bestimmte radioaktive Zerfallsprodukte reichern sich in bestimmten Geweben oder Organen an und können diese nachhaltig schädigen. Die Geräte zum Messen der Intensität der radioaktiven Strahlung des Implantats und der Zerfallsprodukte erfassen ein relativ grosses Gewebevolumen, weshalb die Bestimmung der Durchblutung kleiner Gewebeteile nicht möglich ist. Solche Geräte sind ausserdem nur von einem Fachmann zu bedienen, technisch sehr aufwendig und entsprechend teuer.

Bei einem anderen bekannten Verfahren, das gewöhnlich als Wasserstoff-Auswasch-Verfahren bezeichnet wird, wird das Blut des Probanten mit Wasserstoff angereichert und als Elektrolyt verwendet, der mit zwei in das zu untersuchende Gewebe eingeführten Elektroden ein galvanisches Element bildet. Die elektrische Spannung dieses Elements wird unter anderem von der Konzentration des Wasserstoffs im Blut bestimmt. Bei der Ausführung dieses Verfahrens wird der Wasserstoff mit der Atemluft oder durch Injektion in das Blut eingeleitet. Sobald die Spannung zwischen den Elektroden einen bestimmten Grenzwert erreicht hat, wird die Zufuhr von Wasserstoff unterbrochen und die Abnahme der Spannung als Funktion der Zeit beobachtet. Die Steilheit der Kurve dieser Funktion ist ein Mass für die Duchblutung des Gewebes, bei der das mit Wasserstoff angereicherte Blut abgeführt und durch wasserstofffreies Blut ersetzt wird. Die theoretischen Grundlagen dieses Verfahrens, insbesondere das Berechnen der Potentiale der Elektroden in Abhängigkeit von der Wasserstoff-Ionen-Konzentration mit Hilfe der Nernst'schen Gleichung und der Bestimmung der Durchblutung eines Gewebevolumens aus der Abnahme der Konzentration des Wasserstoffs im Blut mit Hilfe des Fick'schen Gesetzes sind beispielsweise von K. Aukland et al im Circulation Research, Volumen XIV, 1964, Seiten 164ff ausführlich beschrieben. Bei der Ausführung dieses Verfahrens ist keine Implantation und sind keine aufwendigen Apparaturen erforderlich. Ausserdem ermöglicht dieses Verfahren wiederholte Bestimmungen der Durchblutung eines Gewebes bei veränderten Bedingungen und die vergleichsweise kleinen Elektroden ermöglichen das Bestimmen der Durchblutung relativ kleiner Gewebevolumen.

Trotz dieser Vorteile und obwohl die Bestimmung der Durchblutung von Gewebe mit Hilfe inerter Gase bzw. mit Wasserstoff seit mindestens 40 Jahren bekannt und in vielen Publikationen diskutiert ist, blieb die praktische Anwendung dieses Verfahrens bisher auf Tierversuche beschränkt bzw. ist nur eine Messung der Blutflussrate bekannt, die an der Spitze des kleinen Fingers eines Menschen ausgeführt wurde. Die Gründe dafür sind einfach. Mit den bisher zur Ausführung des beschriebenen Verfahrens verwendeten Vorrichtungen konnten auswertbare und reproduzierbare Messungen nur durchgeführt werden, wenn die Stromstärke zwischen den Elektroden und damit auch im Blut bzw. dem zu untersuchenden Gewebe mindestens 1 x 10⁻⁶ Ampere betrug, ein Wert, der für einige Gewebe physiologisch bedenklich oder gar unzulässig ist.

Ein solches Messgerät wird beispielsweise in dem Artikel: "H₂ Clearance Measurement of Blood Flow: A Review of Technique and Polarographic Principles"; von Wise Young, Ph.D., M.D.; in STROKE, Vol. 11, No. 5, September-Oktober 1980, Seiten 552-564, beschrieben.

Der vorliegenden Erfindung lag darum die Aufgabe zugrunde, eine Vorrichtung zu schaffen, die es ermöglicht, das Wasserstoff-Auswasch-Verfahren zur Bestimmung der Durchblutung mit physiologisch unbedenklichen Stromstärken und reproduzierbar durchzuführen.

Erfindungsgemäss wird diese Aufgabe mit einer Vorrichtung der eingangs genannten Art gelöst, die dadurch gekennzeichnet ist, dass die Messelektrode und die Referenzelektrode aus Metallen bestehen, deren chemische Eigenpotentiale nahe beieinanderliegen, sowohl die Messelektrode als auch die Referenzelektrode zur Bildung der Differenz ihrer Potentiale über entsprechende Zuleitungen an den Eingängen des Operationsverstärkers angeschlossen sind, die Messelektrode, die Referenzelektrode und die Neutralelektrode, sowie deren Zuleitungen eine Abschirmung gegen äussere Störfelder aufweisen, zur Erzeugung eines zu induktiv oder kapazitiv eingekoppelten Potentialschwankungen gegenläufigen Potentials im Gewebe, einerseits die Abschirmungen der Mess- und Referenzelektroden und deren Zuleitungen über den positiven Eingang einer Spannungsverstärker- und -umkehrschaltung mit der Neutralekektrode verbunden sind und andererseits der negative Eingang dieser Spannungsverstärker- und -umkehrschaltung mit einem, zur Bildung des Mittelwertes der Potentiale von Mess- und Referenzelektrode, zwischen den Zuleitungen dieser Elektroden angeschlossenen Spannungsteiler, verbunden ist.

Diese erfindungsgemässe Vorrichtung ermöglicht erstmals, die Bestimmung der Durchblutung von Gewebe nach dem vorteilhaften Wasserstoff-Auswasch-Verfahren ohne den für den Menschen physiologisch bedenklichen Messstrom durchzuführen. Die Vorrichtung ist einfach anzuwenden, betriebssicher und kann auch von angelerntem Hilfspersonal bedient werden.

Ein wesentlicher Vorteil dieser Erfindung besteht darin, dass mit diesem Messgerät eine Potentialdifferenzmessung unter physiologisch unbedenklichen Bedingungen vorgenommen wird. Insbesondere erlaubt das erfindungsgemässe Messgerät auch Messungen bei geringen Wasserstoffmengen und in Bereichen, welche normalerweise nur über die Gefässe zugänglich sind, wie beispielsweise in der Herzgegend. Diese bevorzugte Ausführungsform zeichnet sich durch ihre ausgeprägte Humansicherheit und ihre hohe Messempfindlichkeit aus.

Insbesondere dient das Gewebe nur partiell als galvanisches Element. Der durch das Gewebe fliessende Strom ist vernachlässigbar klein und erzeugt insbesondere auch keine die Messung beeinträchtigende Sekundäreffekte durch Ionenverschiebung.

Weitere bevorzugte Merkmale des erfindungsgemässen Blutflussmessgerätes sind in den Ansprüchen angegeben.

Nachfolgend wird die Erfindung anhand eines Ausführungsbeispiels näher erläutert.

Es zeigen:
Fig. 1 ein Schaltbild einer einfachen Ausführungsform des erfindungsgemässen Blutflussmessgerätes,
Fig. 2 ein Schaltbild einer erweiterten Ausführungsform,
Fig. 3 ein Schaltbild einer bevorzugten Ausführungsform, und
Fig. 4a,b,c: Ansichten der Spitze einer Mess-Sonde.

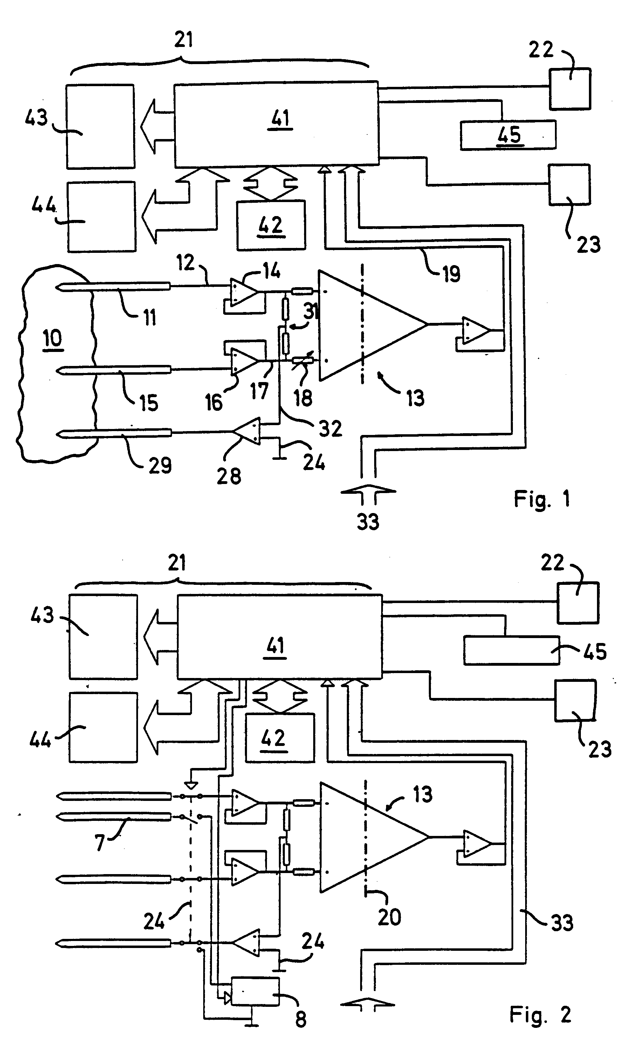

Das in Fig. 1 dargestellte Schaltbild zeigt eine Messelektrode 11, welche in einem auszumessenden Gewebe 10 eingesetzt ist und über eine Zuleitung 12 mit einem ersten Eingang eines strom- und spannungsisolierten Differenzverstärkers 13 verbunden ist. Dieser strom- und spannungsisolierte Differenzverstärker 13 bildet eine potentialgetrennte Schnittstelle resp. galvanische Trennung 20 zwischen dem Datenerfassungsteil und dem Anzeige- und Auswerteteil des Messgerätes. In einer bevorzugten Ausführungsform ist die Messelektrode 11 eine Platinelektrode und wird das Potentialsignal dieser Elektrode 11 über eine Spannungsverstärkerschaltung 14 geleitet. Eine im Gewebe 10 implantierte Referenzelektrode 15 ist über eine Zuleitung 17 mit einem zweiten Eingang des strom- und spannungsisolierten Differenzverstärkers 13 verbunden und besteht in bevorzugten Ausführungsformen aus einer Silberelektrode oder aus einer silberhaltigen, insbesondere einer AgCl-Elektrode. Um den bei solchen galvanischen Elementen fliessenden Strom zu minimalisieren werden die Metalle dieser Elektroden derart gewählt, dass deren chemische Eigenpotentiale E° nahe beieinanderliegen. Insbesondere wird in der bevorzugten Ausführungsform das Signal der Referenzelektrode 15 über einen Spannungsverstärker 16 und für den Nullabgleich über einen regelbaren Widerstand 18 geführt. Dieser Nullabgleich ist wichtig, um lokale Aenderungen der Gewebetemperatur, des pH-Wertes oder andere die Messwerte beeinträchtigende Effekte, beispielsweise Ionentransport bei Iontophorese oder Medikamentenapplikation, abzugleichen.

Der Ausgang des strom- und spannungsisolierten Differenzverstärkers 13 ist über eine Anzeigezuleitung 19 mit einer Anzeigeschaltung 21 verbunden, welche die Messignale mit Hilfe eines A-D-Wandlers (nicht dargestellt) digitalisiert. Diese Anzeigeschaltung 21 umfasst weiterhin geeignete Mittel, um beispielsweise zur Echt-Zeit-Darstellung eines Spannungs-Zeit-Diagramms verschiedene Auswertevorrichtungen 22, 23 zu steuern.

Es versteht sich, dass der Ausgang des strom- und spannungsisolierten Differenzverstärkers 13 direkt mit einem Datenschreiber verbunden werden kann oder zur Auswertung der Messdaten zusätzliche Rechner mit der Anzeigeschaltung 21 verbunden sein können.

Wesentlich für die Humansicherheit des erfindungsgemässen Messgerätes sind die hochohmig ausgelegten Eingänge des strom- und spannungsisolierten Differenzverstärkers 13, welche in der bevorzugten Ausführungsform den Kurzschlussstrom auf 32pA beschränken. Ein geeigneter Differenzverstärker 13 ist der Burr-Braun® 3656, Isolationsverstärker, wie er im Product Data Book der Burr-Braun-Corporation, Juli 1984 näher spezifiziert ist. Insbesondere weist dieser spezielle Elektronikbaustein Eingangswiderstände von 1 x 10¹³ Ohm und eine Spannungsisolation von über 3,5 kV auf.

Die Messempfindlichkeit des vorliegenden Blutflussmessgerätes wird wesentlich erhöht durch Abschirmungen der Zuleitungen 12, 17 der Mess- und Referenzelektroden 11, 15.

Die von äusseren Störfeldern auf diese Abschirmungen induzierten Störspannungen werden mit einer Zuleitung 24 abgegriffen und dem positiven Eingang einer Spannungsverstärker- und -umkehrschaltung 28 zugeführt, deren Ausgang mit einer Neutralelektrode 29 verbunden ist. Der negative Eingang dieser Spannungsverstärker- und -umkehrschaltung 28 ist über eine Zuleitung 32 mit einem Spannungsteiler 31, zur Bildung des Mittelwertes der vom Mess- und Referenzelektrode 11, 15 gemessenen Potentiale, verbunden, welcher Spannungsteiler 31 zwischen den Zuleitungen 12, 17 dieser Elektroden 11, 15 angeschlossen ist. Damit erzeugt die Neutralelektrode 29 ein dem äusseren Störfeld gegenläufiges Potential im auszumessenden Gewebe und kompensiert als aktive Treiberelektrode diese Störpotentiale.

Weitere Ausbildungen des erfindungsgemässen Blutflussmessgerätes liegen im gewöhnlichen technischen Handeln des Fachmanns. So können beispielsweise für die Verbesserung der Messwerte weitere Sonden 33 mit der Anzeigeschaltung 21 direkt verbunden sein. Insbesondere versteht es sich von selbst, weiterführende, zusätzliche Messungen der Gewebetemperatur, pH-Werte, O₂-Partialdruck, O₂-Gehalt etc. oder elektronische Massnahmen zur Glättung und Filterung in der Schaltung zu integrieren.

Ebenso liegt es im Bereich des fachmännischen Könnens, eine Mehrzahl von Mess- resp. Referenzelektroden und eine entsprechende Auswertschaltung zu verwenden, um die gemessenen Daten in dreidimensionaler Darstellung anzuzeigen.

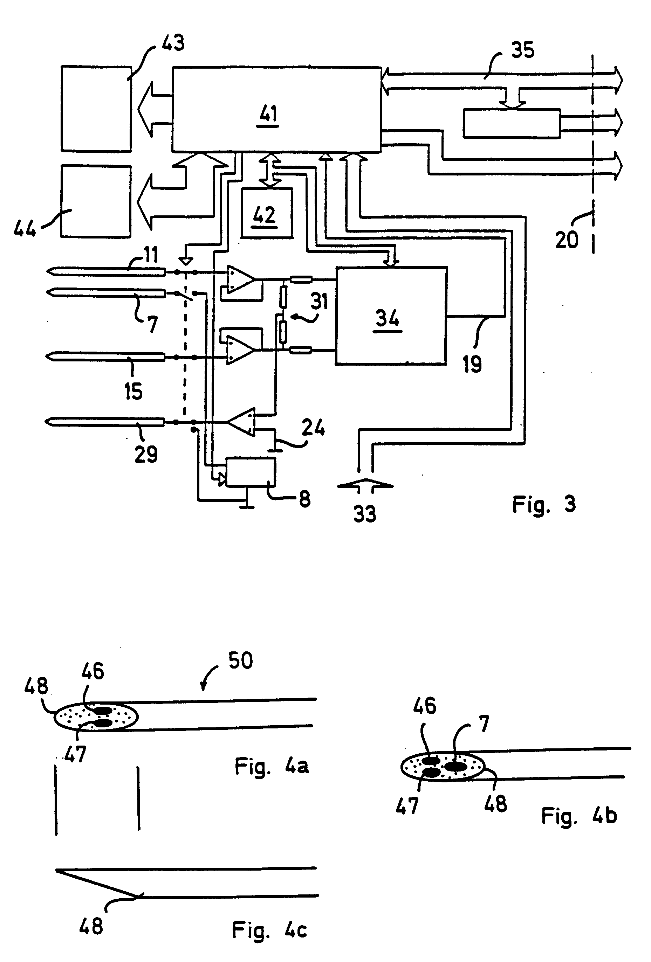

In der in Fig. 3 dargestellten Ausführungsform des erfindungsgemässen Blutflussmessgerätes wird als Operationsverstärker ein handelsüblicher Mikrochip 34 verwendet. Ein für das vorliegende Blutflussmessgerät geeigneter Mikrochip 34 wird beispielsweise im Artikel: "Datenerfassungs-Chip ersetzt 30 Standard-ICs" von G. McGlinchey, et al in Elektronik, 13/22.6.90, Seiten 90-93 beschrieben. Die erfindungswesentliche galvanische Trennung 20 zwischen dem Datenerfassungsteil und dem Auswerteteil des Messgerätes ist in dieser Ausführungsform hinter der Anzeigeschaltung 21 angebracht, während diese potentialgetrennte Schnittstelle 20 in der vorherigen Ausführungsform durch die Verwendung eines strom- und spannungsisolierten Differenzverstärkers 13 vor der Anzeigeschaltung 21 liegt. Im Unterschied zum ersten Ausführungsbeispiel werden die Ausgangssignale über einen Opto-Bus 35 geführt, welcher in natürlicher Weise die gewünschte galvanische Trennung schafft.

Eine sowohl in Fig. 2 als auch in Fig. 3 dargestellte Weiterbildung des erfindungsgemässen Messgerätes betrifft dessen Verwendung zur aktiven Elektrolyse, bei welcher aktiv mit einer zusätzlichen Elektrode 7, insbesondere einer Platinelektrode, der H₂-Gehalt des durchbluteten Gewebes lokal erhöht wird. Der Vorteil dieser Weiterbildung ist unmittelbar deutlich. Einerseits kann damit unabhängig von einer Inhalation, beispielsweise mit Hilfe eines H₂-Spray, eine ausreichend hohe Wasserstoffkonzentration in dem zu messenden Bereich erzeugt werden und können andererseits die Elektrolysezeit und der Elekrolysestrom selbst als Mass für die Wasserstoffkonzentration verwendet werden. Bei dieser Erweiterung wird die zusätzliche Elektrode 7 an eine Konstantstromquelle 8 geschaltet, welche ausgelegt ist, um Ströme von 0 bis 50 »A liefern zu können und welche mit dem Mikroprozessor 41 der Anzeigeschaltung 21 verbunden ist.

Diese Anzeigeschaltung 21 umfasst im weiteren einen Speicher 42, einen Anzeigemodul 43 und eine Tastatur 44. In einer erprobten Ausführungsform sind die einzelnen Messelektroden zu einer Mess-Sonde zusammengebaut, welche lösbar mit einem abgeschirmten Messkanalkabel verbunden ist. Die Mess-Sonde 50 ist nadelförmig ausgebildet und weist eine, wie in den Fig. 4a, 4b und 4c dargestellt, abgeschrägte Spitze auf. Einfacherweise besteht die Mess-Sonde aus einer hohlen Edelstahlnadel 48, in welche die Silber- 46 und Platinelektroden 47 eingebaut sind. Die Edelstahlnadel 48 dient somit gleichzeitig als Abschirmung und als Sondenträger. Es versteht sich, dass geeignete Mittel vorgesehen sind, um die einzelnen Elektroden gegeneinander zu isolieren und zu haltern, insbesondere durch Lackbeschichtungen und Giessharze. Fig. 4b zeigt eine Sondenspitze für die Verwendung als aktive Elektrolyse-Sonde. Dafür ist eine zusätzliche Elektrode 7 vorgesehen, welche mit den anderen Elektroden auf der abgeschrägten Nadelfläche ein geometrisch genau definiertes galvanisches Element bildet. Gleichzeitig oder im Anschluss an diese lokale Elektrolyse kann mit den anderen Elektroden gemessen werden. Es versteht sich, dass die Mess-Sonde über eine lösbare Steckverbindung, beispielsweise einen Bajonettoder Schnappverschluss, mit dem Messgerät verbunden ist, insbesondere da solche Messungen zur Beobachtung über einen längeren Zeitraum vorgenommen werden können und damit nicht für jede einzelne Messung die Sonde neu plaziert werden muss. Es versteht sich auch, dass das erfindungsgemässe Messgerät mit mehreren Messkreisen für mehrere Mess-Sonden ausgestattet sein kann. Die für die Auswertung der abgelesenen Messwerte verwendeten Geräte 22, 23 sind an entsprechenden pheripheren Schnittstellen 45 angeschlossen. Insbesondere umfasst das vorliegende Messgerät Analogausgänge für einen XY-Schreiber, Digitalanzeigen und Messindikationsleuchten.

Die für die Auswertung der Messignale verwendete Software stützt sich auf das Fick'sche Gesetz ab, welches die Aenderung der Gaskonzentration als Funktion des Blutflusses beschreibt, und wird im folgenden nicht näher erläutert. Literatur dazu und andere mathematische Auswertmethoden sind dem Fachmann hinlänglich bekannt.

Erweiterungen des vorliegenden Messgerätes, beispielsweise durch einen PC-Monitor, Vorrichtungen zum Geräteschutz, Sondenhalterungen liegen im Bereich fachmännischen Könnens. Ebenso können auch für die Sonden verwendete Materialien geeignet gewählt werden, insbesondere kann der Elektrodenträger aus ausgesuchten Kunststoffen gefertigt sein.

Für den Fachmann selbstverständlich ist auch die Verwendung des vorliegenden Blutflussmessgerätes für Blutflussmessungen, bei welchen nicht Wasserstoff inhaliert, erzeugt oder injiziert wird, sondern bei welchen Ascorbinsäure, d.h. Vitamin C, oder andere Redox-Substanzen, wie sie aus der Indikator-Dilutions-Technik bekannt sind, verabreicht werden. Die für die Auswertung dieser Messverfahren geeigneten Algorithmen sind in Fachkreisen hinreichend bekannt.

Das oben beschriebene Blutflussmessgerät kann für jedes durchblutete oder von einer anderen Körperflüssigkeit durchflossene oder umspülte Organ verwendet werden und wird als besonders geeignet erachtet bei der Kreislaufzeitenmessung, Shunt-Messung, Herzvolumenmessung, Liquorzirkulationsmessung, Infusionsvolumenmessung, Gefässdurchflussvolumenmessung, Diffusionsvolumen- und Konzentrationsmessung, Urinausscheidungsmessung, Tränenflüssigkeitsmessung, in der Transplantations- und Implantationschirurgie, plastischen Chirurgie, Tumorchirurgie, Neurochirurgie, Radiotherapie, Parodonologie, Endodontie, bei der Medikamentenwirkung und Dosierung, Höhen- und Tiefenphysiologie, zur Beurteilung von Akupunktureffekten und anderem mehr.

Es versteht sich, dass das erfindungsgemässe Messgerät nicht nur zur Diagnostik und Therapieverlaufskontrolle im medizinischen Bereich, sondern auch in Bereichen der allgemeinen Strömungstechnik verwendet werden kann.

## Patentansprüche

1. Blutflussmessgerät nach dem Wasserstoff-Auswasch-Verfahren mit einer Messelektrode (11), einer Referenzelektrode (15) und einer Neutralelektrode (29), sowie mit einem Operationsverstärker (13) und mit einer Signalanzeigevorrichtung (21, 22, 23), wobei die Messelektrode (11) und die Referenzelektrode (15) aus Metallen bestehen, deren chemische Eigenpotentiale nahe beieinanderliegen,
sowohl die Messelektrode (11) als auch die Referenzelektrode (15) zur Bildung der Differenz ihrer Potentiale über entsprechende Zuleitungen (12, 17) an den Eingängen des Operationsverstärkers (13) angeschlossen sind,
die Messelektrode (11), die Referenzelektrode (15) und die Neutralelektrode (29) sowie deren Zuleitungen (12, 17) eine Abschirmung (24) gegen äussere Störfelder aufweisen,
zur Erzeugung eines zu induktiv oder kapazitiv eingekoppelten Potentialschwankungen gegenläufigen Potentials im Gewebe (10), einerseits die Abschirmungen der Mess- und Referenzelektroden (11, 15) und deren Zuleitungen (12, 17) über den positiven Eingang einer Spannungsverstärker- und -umkehrschaltung (28) mit der Neutralelektrode (29) verbunden sind und andererseits der negative Eingang dieser Spannungsverstärker- und -umkehrschaltung (28) mit einem, zur Bildung des Mittelwertes der Potentiale von Mess- und Referenzelektrode (11, 15), zwischen den Zuleitungen (12, 17) dieser Elektroden angeschlossenen Spannungteiler (31), verbunden ist.

2. Blutflussmessgerät gemäss Anspruch 1, dadurch gekennzeichnet, dass der Operationsverstärker (13) ein strom- und spannungsisolierter Differenzverstärker mit hochohmigen Eingängen ist,

3. Blutflussmessgerät gemäss Anspruch 2, dadurch gekennzeichnet, dass der Differenzverstärker ein integrierter, transformatorgekoppelter Isolationsverstärker mit einem Eingangswiderstand von mindestens 1 x 10¹³ Ohm ist.

4. Blutflussmessgerät gemäss Anspurch 1, dadurch gekennzeichnt, dass der Operationsverstärker (13) einen Mikrochip (34) mit einem rauscharmen, bipolaren Präzisionsverstärker umfasst und für die galvanische Trennung zwischen dem Mikrochip (34) und einer Auswertevorrichtung (22, 23) ein Opto-Bus (35) vorgesehen ist.

5. Blutflussmessgerät gemäss Anspruch 1, dadurch gekennzeichnet, dass eine Elektrolyse-Elektrode (7) mit einer Konstantstromquelle (8) einschaltbar verbunden ist.

6. Blutflussmessgerät gemäss Anspruch 1, dadurch gekennzeichnet, dass die Elektroden in einer nadelförmigen Mess-Sonde gehaltert sind und diese Mess-Sonde lösbar an einem Messkanalkabel befestigt ist.

7. Blutflussmessgerät gemäss Anspruch 6, dadurch gekennzeichnet, dass das Blutflussmessgerät mindestens eine Mess-Sonde und mindestens eine Zusatz-Sonde (33) für die Bestimmung zusätzlicher Mess-Parameter umfasst.

8. Mess-Sonde für ein Blutflussmessgerät nach Anspruch 6.

9. Verwendung eines Blutflussmessgerätes nach Anspruch 1 in der Wasserstoff-Dilutionstechnik.

10. Verwendung eines Blutflussmessgerätes gemäss Anspruch 1, in der Ascorbinsäure-Dilutions-Technik.

11. Verwendung eines Blutflussmessgerätes gemäss Anspruch 1 in der allgemeinen Strömungstechnik.

## Claims

1. Blood flow measurement apparatus operating according to the H₂ clearance method having a measuring electrode (11), a reference electrode (15) and a neutral electrode (29), an operational amplifier (19) and having a signal display device (21, 22, 23), whereby the measuring electrode (11) and the reference electrode (15) are formed of metals whose natural chemical potentials lie close to one another, both the measuring electrode (11) and the reference electrode (15) being connected to corresponding input lines (12, 17) at the entrances of the operational amplifier (19) in order to form the difference of their respective potentials,
the measuring electrode (11), the reference electrode (15) and the neutral electrode (29), as well as their corresponding input lines (12, 17) being provided with a shield (24) to safeguard against exterior jamming fields,
the shielding of the measuring and reference electrodes (11, 15) and their corresponding input lines (12, 17) are connected via the positive entrance of a voltage amplifying and inverting circuit (28) with the neutral electrode (29) on the one hand, and the negative entrance of this voltage amplifying and inverting circuit (28) is connected with a voltage divider in order to form an average value of the potentials of the measuring and reference elctrode (11, 15) on the other hand, for the purpose of generating a potential in the tissue (19) which is opposite to inductive or capacitive coupled-in potential fluctuations.

2. Blood flow measuring apparatus according to claim 1, characterized in that the operational amplifier (13) comprises a current and voltage isolated differential amplifier having high-ohm inputs.

3. Blood flow measuring apparatus according to claim 2, characterized in that the differential amplifier comprises an integrated transformer coupled isolation amplifier having an input resistance of at least 1 x 10¹³ ohm.

4. Blood flow measuring apparatus according to claim 1, characterized in that the operational amplifier (13) comprises a microchip (34) having a substantially noise free, bipolar precision amplifier, and an opto-bus (35) being provided for the galvanic isolation between the microchip (34) and an evaluation means (22, 23).

5. Blood flow measuring apparatus according to claim 1, characterized in that an electrolysis electrode (7) is selectively connected with a constant current source (8).

6. Blood flow measuring apparatus according to claim 1, characterized in that the electrodes are mounted in a needle-shaped measuring probe, said measuring probe being releasably connected with a measuring channel cable.

7. Blood flow measuring apparatus according to claim 6, characterized in that the blood flow measuring apparatus comprises at least one measuring probe and at least one additional probe (33) for the determination of additional measuring parameters.

8. Measuring probe for a blood flow measuring apparatus according to claim 6.

9. Use of a blood flow measuring apparatus according to claim 1 in hydrogen-dilution technology.

10. Use of a blood flow measuring apparatus according to claim 1 in ascorbic acid-dilution technology.

11. Use of a blood flow measuring apparatus according to claim 1 in general industrial hydrodynamic technology.

## Revendications

1. Dispositif de mesure de débit sanguin selon le procédé d'échange d'hydrogène comportant une électrode de mesure (11), une électrode de référence (15) et une électrode neutre (29), ainsi qu'un amplificateur opérationnel (13) et un dispositif de signalisation (21, 22, 23),
dans lequel l'électrode de mesure (11) et l'électrode de référence (15) sont réalisées dans des métaux dont les potentiels chimiques sont proches l'un de l'autre,
l'électrode de mesure (11) ainsi que l'électrode de référence (15) sont raccordées par des lignes d'arrivée (12, 17) respectives aux entrées de l'amplificateur opérationnel (19), en vue de former la différence de leurs potentiels, l'électrode de mesure (11), l'électrode de référence (15) et l'électrode neutre (29) ainsi que leurs lignes d'arrivée (12, 17) comportent un blindage (24) à l'égard de champs de perturbations extérieurs,
en vue de produire dans le tissu (10) un potentiel de sens contraire aux fluctuations de potentiel introduites de manière inductive ou capacitive, d'une part, les blindages des électrodes de mesure et de référence (11, 15) et leurs lignes d'arrivée (12, 17) sont reliées à l'électrode neutre (29), par l'entrée positive d'un circuit amplificateur et inverseur de tension (28) et, d'autre part, l'entrée négative de ce circuit amplificateur et inverseur de tension (28) est reliée à un diviseur de tension (31), raccordé entre les lignes d'arrivée (12, 17) des électrodes de mesure et de référence (11, 15), pour former la valeur moyenne des potentiels de ces électrodes.

2. Dispositif de mesure de débit sanguin selon la revendication 1, caractérisé en ce que l'amplificateur opérationnel (13) est un amplificateur de différence isolé en intensité et en tension avec des entrées de valeur ohmique élevée.

3. Dispositif de mesure de débit sanguin selon la revendication 2, caractérisé en ce que l'amplificateur de différence est un amplificateur d'isolation intégré, couplé en transformateur avec une résistance d'entrée d'au moins 1 x 10¹³ ohms.

4. Dispositif de mesure de débit sanguin selon la revendication 1, caractérisé en ce que l'amplificateur opérationnel (13) comporte une microplaquette (34) avec un amplificateur de précision bipolaire à faible bruit et pour la séparation galvanique entre la microplaquette (34) et un dispositif d'évaluation (22, 23), il est prévu un bus optique (35).

5. Dispositif de mesure de débit sanguin selon la revendication 1, caractérisé en ce qu'une électrode d'électrolyse (7) est reliée de manière commutable avec une source de courant constant (8).

6. Dispositif de mesure de débit sanguin selon la revendication 1, caractérisé en ce que les électrodes sont maintenues dans une sonde de mesure en forme d'aiguille et cette sonde de mesure peut être fixée de manière amovible sur un câble de canal de mesure.

7. Dispositif de mesure de débit sanguin selon la revendication 6, caractérisé en ce que le dispositif de mesure de débit sanguin comporte au moins une sonde de mesure et au moins une sonde supplémentaire (33) pour déterminer des paramètres de mesure supplémentaires.

8. Sonde de mesure pour un dispositif de mesure de débit sanguin selon la revendication 6.

9. Utilisation d'un dispositif de mesure de débit sanguin selon la revendication 1, en technique de dilution d'hydrogène.

10. Utilisation d'un dispositif de mesure de débit sanguin selon la revendication 1, en technique de dilution d'acide ascorbique.

11. Utilisation d'un dispositif de mesure de débit sanguin selon la revendication 1, en technique générale des fluides.
